# EUROPEAN PATENT SPECIFICATION

(11) **EP 3 442 435 B1**
(45) Date of publication and mention of the grant of the patent: **25.03.2020**
(21) Application number: 17717705.2
(22) Date of filing: 13.04.2017
(51) Int. Cl.: A61B 17/02, A61G 13/12, A61B 17/326

(54) **PENILE SURGICAL SEPARATOR-FIXATOR**
CHIRURGISCHER PENILER SEPARATOR-FIXATOR
SÉPARATEUR-FIXATEUR CHIRURGICAL PÉNIEN

(30) Priority: 15.04.2016 ES 201630469 U
(43) Date of publication of application: 20.02.2019
(73) Proprietor: Fundació Institut d'Investigació en Ciències de la Salut Germans Trias i Pujol, 08916 Badalona (ES)
(72) Inventor: DE DIEGO SUAREZ, Marta, 08019 BARCELONA (ES); ISNARD BLANCHAR, Rosa Maria, 08012 BARCELONA (ES)
(74) Representative: ZBM Patents - Zea, Barlocci & Markvardsen
(86) International application number: PCT/EP2017/058986
(87) International publication number: WO 2017/178613

(56) References cited:
- ES-U- 1 060 574
- US-A1- 2012 157 763

## Description

This application claims the benefit of Spanish utility model application no. 201630469, publication number ES1157458, filed 15 April 2016.

The present disclosure relates to a penile surgical separator-fixator, whose obvious purpose is to maintain the penis properly supported and held during surgeries of the urethra or of the rest of penile structures.

### BACKGROUND

In any surgery of penile urethra, it is required for the surgeon to keep the penis resting on its dorsal side most of the surgical time, and hold it immobile enough to be able to work in a comfortable way.

This arrangement was classically achieved by traversing the glans with a stitch and holding both ends of the thread with a surgical instrument, said thread being pulled by the corresponding assistant.

With such fastening, besides disabling one hand of the professional, a constant pulling force is not maintained, as it depends on the skill and fatigue of the assistant.

Sometimes the instrument for fixing the thread is also fixed to the surgical field, such as sterile clothing or paper that delimit and isolate the surgical field, although this system also does not maintain the desired pulling force.

Although suitable penile holding devices are not known, a metallic annular device has recently emerged consisting of a readjustment of the same system used for other surgical areas (ano-rectal ....) although since the device is circumferential and a part thereof is in the ventral area of the penis, it interferes in that area with the surgeon's own hands, affecting comfort.

Congenital malformations of the urethra requiring surgery in children are common. There is also surgery of the urethra in adults, due to acquired problems. Less common are the surgeries of the back of the penis, although the immobility of the tissues is still required.

With respect to surgery in children, surgical correction of malformations is usually performed early, and often in the first year of life in children, so that the size complicates surgery even more and makes it more necessary that the tissue to be treated is held immobile and with suitable tension.

The surgical field, already small, is even more in the above mentioned cases, so that tooling to be used has to be the least uncomfortable and most discreet possible.

Document ES1060574U discloses a prior art separator for surgical operations.

### SUMMARY

The device disclosed herein solves the above described problem in a fully satisfactory manner, based on a simple but highly efficient structure, providing a device or means that does not disturb the surgeon during the surgical intervention and allows the intervention to be performed comfortably.

For this purpose, and more specifically, the separator according to this disclosure comprises a plate, determining a smooth and hard surface, intended to rest on the patient's abdominal area, such that the material from which the plate forming the separator-fixator is made should be appropriate so as not to be harmful to the patient's skin. In the less common cases of surgery on the back of the penis, the device works in the same way, using the scrotal area as support, and arranged in a specular manner with respect to the latter.

The plate may have a slight curvature to adapt to the curvature of the abdominal area of the patient, such as to offer a more stable support.

One of the main characteristics of the plate concerned is that a curved recess is formed at one corresponding side thereof, with a configuration corresponding to the contour of the base of the penis, so that the penis is placed on said recess and supported on the plate, preventing it from slipping.

The curved recess may have a smooth edge to provide more comfort and prevent damage to the user. The edge of the plate itself may be smoothened or rounded in the area of the curved recess, for example by sanding or polishing. An additional part may also be provided in the area of the curved recess, such as a strip of silicone or other soft material attached around the edge of the plate, for example with an adhesive.

In addition, the plate has a series of holes distributed in an area opposite to the recess, for the passage of the threads that previously have to hold the glans of the penis, so that each end of the thread is passed through one of the holes of the opposite end of the recess in the base of the penis, and it is fixed to the rear side of the plate with the same instrument which typically held the thread in conventional surgery. It also comprises transverse grooves on the rest of the sides of the plate for the passage of different traction threads of other structures of the surgical field, at the convenience of the surgeon. They may also be used for the passage of the threads of the glans, if the surgeon prefers the grooves instead of the holes.

According to these characteristics, by resting the plate on the patient's abdomen, the resting of the penis on the recess and the pulling force that is performed by the thread, the tension of the tissues can be maintained without having to make other type of fixations.

The pulling force can be adjusted simply by loosening or tightening the threads, while the linear grooves allow traction threads to be fixed to other parts of the penis, such as the foreskin, for better exposure to the surgical field.

By means of the thus constituted separator, it is possible to perform the classic clamping of the base of the penis without any difficulty, to operate with ischemia and to prevent bleeding, in a comfortable way, since the base of the penis is free, and since it is not fixed in any way to the body, as it simply rests thereon, the penis and the separator can be positioned vertically at any time without any difficulty.

Finally, the plate constituting the separator-fixator can be applied and removed as many times as necessary by simply unclamping the thread on the back of the plate constituting the separator-fixator.

The device constituting the separator-fixator is useful even in surgery with the urethra at the base of the penis, with complex surgeries of the urethra being able to be performed in a simple way and without the need for the assistant pulling the penis.

Finally, it should be pointed out that the separator-fixator shall be placed on the market in two sizes, for children's penises and for adult's penises, since the length of the separator allows working with a fairly wide range of penile lengths.

The separator-fixator may be a disposable or single-use device, for example made of a stiff plastic material. It may also be a re-usable device, for example made of metal, such as to be sterilisable.

### DESCRIPTION OF THE DRAWINGS

In order to complement the description which will be given below and in order to aid in a better understanding of the features of the present disclosure, according to a preferred example of practical embodiment thereof, a set of drawings are attached herein as part of the description in which the following is illustrated in an illustrative and non-limiting character:

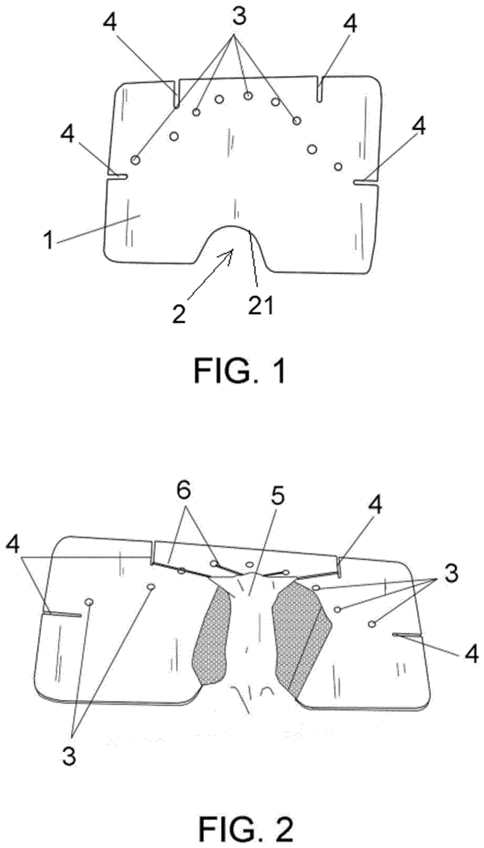

Figure 1 shows a representation corresponding to a perspective view of a penile surgical separator-fixator made according to the object of the present disclosure.
Figure 2 shows a perspective view of the form of use of said device in urethral surgery.

### DESCRIPTION OF EMBODIMENTS

As it can be seen from the above figures, the separator-fixator of the present disclosure comprises a plate (1), made of any suitable material which is not harmful to the user's skin, the plate (1) being of a hard type and preferably with a degree of curvature such as to better adapt to the shape of the abdominal area of the patient on which it is intended to rest, and including a rounded recess (2) in correspondence with one of its edges, and a number of holes (3), as well as linear grooves (4).

When a surgical intervention of the urethra is performed by a surgeon, the penis (5) rests on the recess (2), perfectly positioned resting on the surface of the plate (1), while the immobilization of said penis (5) is carried out by threads (6) passing through the glans itself and passing selectively through the holes (3) and/or grooves (4) to be knotted at the bottom or rear side of the plate (1), thereby achieving a perfect immobilization of the penis (5) so that the surgeon can comfortably work in a surgical intervention of urethra. The grooves (4) also allow the fixation of other traction points to the surgical field.

Hence, each end of the thread (6) is passed through one of the holes (3) on the end that is opposite to the recess (2) for the base of the penis (5), and it is fixed on the opposing side of the plate (1) itself, using the same instrument that is normally used for holding threads in conventional surgery, in such a way that the arrangement and attachment of the threads (6) will maintain the tension of the tissues without having to provide fixations elsewhere, while the linear grooves (4) allow traction threads of other parts of the penis (5) to be fixed for a better exposure of the surgical field.

It is also possible to provide the separator with a clamping system on its rear side, for each end of the thread, thus making it unnecessary to use the fixation instrument that is commonly used.

The curved recess (2) may have a smooth edge (21) to provide more comfort and prevent damage to the user. The edge of the plate itself in the area of the curved recess (2) may be smoothened, for example by sanding or polishing. Alternatively, or additionally, an additional part (not shown in the figures) may be provided in the area of the curved recess (2), such as a strip of silicone or other soft material attached around the edge of the plate, for example with an adhesive.

The plate (1) may be a disposable, single-use part, or it may be re-usable. If it is reusable it may be made of metal, for example stainless steel, or of a sterilisable plastic. If it is made disposable, it may be of a more economic plastic material.

## Claims

1. Penile surgical separator-fixator, suitable for the immobilization of the penis during a surgical intervention of the urethra, **characterized in that** it comprises a smooth, hard plate (1), made of a material that is not damaging for the user's skin, the plate (1) having in correspondence with one of its edges a curved recess (2) to support the base of the patient's penis, whereas opposite to said recess (2) a plurality of holes (3) and grooves (4) are provided for the passage of threads for immobilization of the penis during surgery.

2. Penile surgical separator-fixator, suitable for the immobilization of the penis during a surgical intervention of the urethra, as claimed in the preceding claim, comprising a clamping system on a rear side of the plate (1) for fixing the threads.

3. Penile surgical separator-fixator, suitable for the immobilization of the penis during a surgical intervention of the urethra, as claimed in any of the preceding claims, wherein the grooves (4) are transverse grooves.

4. Penile surgical separator-fixator, suitable for the immobilization of the penis during a surgical intervention of the urethra, as claimed in any of the preceding claims, wherein the grooves (4) are linear grooves.

5. Penile surgical separator-fixator, suitable for the immobilization of the penis during a surgical intervention of the urethra, according to any of the preceding claims, wherein the edge (21) of the plate is smoothened or rounded in the area of the curved recess (2).

6. Penile surgical separator-fixator, suitable for the immobilization of the penis during a surgical intervention of the urethra, according to any of the preceding claims, comprising a strip of soft material attached around the edge (21) of the plate in the area of the curved recess (2).

7. Penile surgical separator-fixator, suitable for the immobilization of the penis during a surgical intervention of the urethra, according to any of the preceding claims, wherein the plate has a curvature to adapt to the abdominal area of the patient.

## Patentansprüche

1. Chirurgische Vorrichtung zur Separation und Fixierung des Penis, die geeignet zur Immobilisierung des Penis während eines chirurgischen Eingriffs der Urethra ist, **dadurch gekennzeichnet, dass** sie eine glatte, harte Platte (1) umfasst, die aus einem Material besteht, welches unschädlich für die Haut des Benutzers ist, wobei die Platte (1) eine gekrümmte Aussparung (2) entsprechend einer deren Kanten hat, um die Basis des Patientenpenis zu stützen, während eine Vielzahl von Löchern (3) und Schlitzen (4) für den Durchgang von Fäden zur Immobilisierung des Penis während der Chirurgie gegenüber der Aussparung (2) vorhanden sind.

2. Chirurgische Vorrichtung zur Separation und Fixierung des Penis, die geeignet zur Immobilisierung des Penis während eines chirurgischen Eingriffs der Urethra ist, wie im vorhergehenden Anspruch beansprucht, umfassend ein Klemmsystem an einer Rückseite der Platte (1) zur Fixierung der Fäden.

3. Chirurgische Vorrichtung zur Separation und Fixierung des Penis, die geeignet zur Immobilisierung des Penis während eines chirurgischen Eingriffs der Urethra ist, wie in einem der vorhergehenden Ansprüche beansprucht, wobei die Schlitze (4) querlaufende Schlitze sind.

4. Chirurgische Vorrichtung zur Separation und Fixierung des Penis, die geeignet zur Immobilisierung des Penis während eines chirurgischen Eingriffs der Urethra ist, wie in einem der vorhergehenden Ansprüche beansprucht, wobei die Schlitze (4) lineare Schlitze sind.

5. Chirurgische Vorrichtung zur Separation und Fixierung des Penis, die geeignet zur Immobilisierung des Penis während eines chirurgischen Eingriffs der Urethra ist, nach einem der vorhergehenden Ansprüche, wobei die Kante (21) der Platte im Bereich der gekrümmten Aussparung (2) geglättet oder abgerundet ist.

6. Chirurgische Vorrichtung zur Separation und Fixierung des Penis, die geeignet zur Immobilisierung des Penis während eines chirurgischen Eingriffs der Urethra ist, nach einem der vorhergehenden Ansprüche, umfassend einen Streifen aus einem weichen Material, der um die Kante (21) der Platte herum im Bereich der gekrümmten Aussparung (2) befestigt ist.

7. Chirurgische Vorrichtung zur Separation und Fixierung des Penis, die geeignet zur Immobilisierung des Penis während eines chirurgischen Eingriffs der Urethra ist, nach einem der vorhergehenden Ansprüche, wobei die Platte eine Krümmung zur Anpassung zum abdominalen Bereich des Patienten hat.

## Revendications

1. Séparateur-fixateur chirurgical pénien, approprié pour l'immobilisation du pénis pendant une intervention chirurgicale de l'urètre, **caractérisé en ce qu'**il comprend une plaque dure et lisse (1), faite en un matériau qui n'endommage pas la peau de l'utilisateur, la plaque (1) ayant, en correspondance avec l'un de ses bords, un évidement incurvé (2) pour soutenir la base du pénis du patient, alors que à l'opposé dudit évidement (2) une pluralité de trous (3) et de rainures (4) sont disposés pour le passage de fils pour l'immobilisation du pénis pendant la chirurgie.

2. Séparateur-fixateur chirurgical pénien, approprié pour l'immobilisation du pénis pendant une intervention chirurgicale de l'urètre, tel que revendiqué dans la revendication précédente, comprenant un système de serrage sur un côté arrière de la plaque (1) pour fixer les fils.

3. Séparateur-fixateur chirurgical pénien, approprié pour l'immobilisation du pénis pendant une intervention chirurgicale de l'urètre, tel que revendiqué dans l'une quelconque des revendications précédentes, dans lequel les rainures (4) sont des rainures transversales.

4. Séparateur-fixateur chirurgical pénien, approprié pour l'immobilisation du pénis pendant une intervention chirurgicale de l'urètre, tel que revendiqué dans l'une quelconque des revendications précédentes, dans lequel les rainures (4) sont des rainures linéales.

5. Séparateur-fixateur chirurgical pénien, approprié pour l'immobilisation du pénis pendant une intervention chirurgicale de l'urètre, selon l'une quelconque des revendications précédentes, dans lequel le bord (21) de la plaque est lissé ou arrondi dans la région de l'évidement incurvé (2).

6. Séparateur-fixateur chirurgical pénien, approprié pour l'immobilisation du pénis pendant une intervention chirurgicale de l'urètre, selon l'une quelconque des revendications précédentes, comprenant une bande de matériau doux attachée autour du bord (21) de la plaque dans la région de l'évidement incurvé (2).

7. Séparateur-fixateur chirurgical pénien, approprié pour l'immobilisation du pénis pendant une intervention chirurgicale de l'urètre, selon l'une quelconque des revendications précédentes, dans lequel la plaque a une courbure pour son adaptation à la région abdominale du patient.
